(19) Europäisches Patentamt
European Patent Office
Office européen des brevets

(11) EP 1 552 837 A1

(12) EUROPEAN PATENT APPLICATION
published in accordance with Art. 158(3) EPC

(43) Date of publication:
**13.07.2005 Bulletin 2005/28**

(21) Application number: **03766702.9**

(22) Date of filing: **01.08.2003**

(51) Int Cl.[7]: **A61K 31/59**, A61P 17/06, A61P 43/00

(86) International application number:
**PCT/JP2003/009814**

(87) International publication number:
**WO 2004/012743 (12.02.2004 Gazette 2004/07)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **01.08.2002 JP 2002224297**

(71) Applicant: **CHUGAI SEIYAKU KABUSHIKI KAISHA**
**Tokyo, 115-8543 (JP)**

(72) Inventor: **SHIMAOKA, Shin,**
**Chugai Seiyaku Kabushiki Kaisha**
**Gotenba-shi, Shizuoka 412-8 513 (JP)**

(74) Representative: **VOSSIUS & PARTNER**
**Siebertstrasse 4**
**81675 München (DE)**

# (54) ANTIPSORIATIC AGENT

(57) An object of the present invention is to synthesize a pharmaceutical effective for the treatment of psoriasis.

A therapeutic agent for psoriasis, comprising 2β-(3-hydroxypropyloxy)-1α,25-dihydroxyvitamin $D_3$ as an active ingredient, is provided by the present invention.



Printed by Jouve, 75001 PARIS (FR)

## Description

### TECHNICAL FIELD

This invention relates to a therapeutic agent for psoriasis, comprising a vitamin D derivative as an active ingredient.

### BACKGROUND ART

Psoriasis is a chronic intractable skin disease, characterized by abnormal proliferation of skin cells. Its etiology is not yet clear, but the deviation of skin cells from the normal growth mechanism and differentiation mechanism is considered to be a main cause. There has been an increase in the number of cases of psoriasis in recent years, and most psoriatic cases involve well-demarcated papules or erythemas with thick scales, and follow a chronic course. This type of psoriasis is called psoriasis vulgaris. Unlike psoriasis vulgaris, psoriasis pustulosa forms pustules on erythemas. Psoriasis pustulosa is classified into generalized (Zumbush's) pustular psoriasis which occurs over wide areas and involves systemic symptoms, and localized (Barber's) pustular psoriasis which develops over small areas, such as the hands or feet. Psoriasis may occasionally cause redness, swelling, degeneration or ankylosis of joints of the hands or feet, elbow and knee. This is called arthritic psoriasis.

Treatments for psoriasis include external application of corticosteroids, photochemotherapy (PUVA), and oral administration of retinoids. However, these treatments have not always had a satisfactory therapeutic effect. In recent years, 1α,25-dihydroxyvitamin $D_3$, calcipotriol, etc., which are known as active vitamin $D_3$, have been shown to have the activity of suppressing the proliferation of keratinocytes, and to be useful as therapeutic agents for psoriasis (European Patent Publication No. 129003, "Vitamin D in Dermatology", edited by Knud Kragballe (2000), Marcel Dekker Inc., and Drugs 43(3), 415-429 (1992)). However, more potent and more effective pharmaceuticals are still desired.

2β-(3-Hydroxypropyloxy)-1α,25-dihydroxyvitamin $D_3$, which is a vitamin D derivative having a substituent at the 2-position, is known to have a calcium regulating action (JP 61-267549 A) and an osseous union promoting action (JP 08-12580 A). However, its use as a therapeutic agent for psoriasis has not been known at all.

### DISCLOSURE OF THE INVENTION

As described above, existing treatment methods and therapeutic agents for psoriasis have not been entirely satisfactory, and more potent and effective treatments and therapeutic drugs have been desired. It is an object of the present invention to provide an effective therapeutic agent and treating method for psoriasis.

The inventor of the present invention investigated the suppressing action of 2β-(3-hydroxypropyloxy)-1α, 25-dihydroxyvitamin $D_3$ on the keratinocyte proliferation. The inventor has found that surprisingly this compound has a very potent suppressing action, in comparison with active vitamin $D_3$, and has accomplished the present invention.

That is, the present invention provides a therapeutic agent for psoriasis, which agent comprises a compound represented by the following Formula (I)

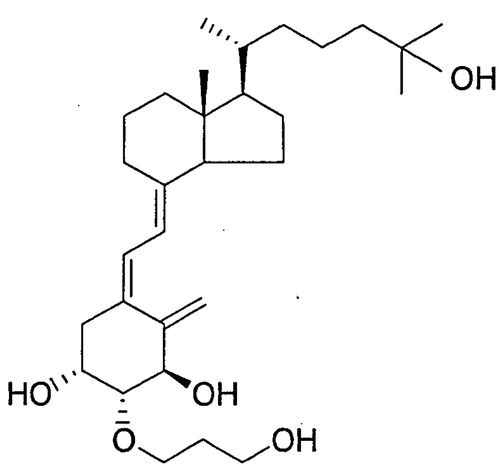

as an active ingredient.

The therapeutic agent for psoriasis according to the present invention preferably suppresses the proliferation of keratinocytes.

The therapeutic agent for psoriasis according to the present invention may be administered to animals as well as humans.

According to another aspect of the present invention, there is provided a method for the treatment of psoriasis in a human or an animal, the method comprises administering a therapeutically effective amount of a compound represented by the following Formula (I)

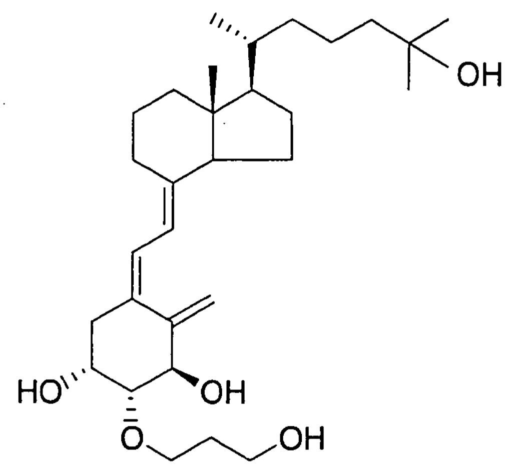

to a human or an animal in need of such treatment.

According to still another aspect of the present invention, there is provided use of a compound represented by the following Formula (I)

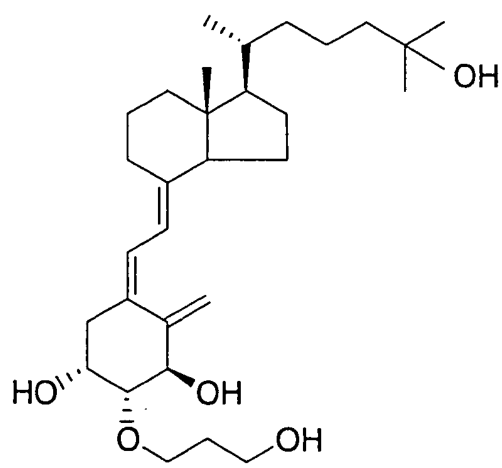

in production of a therapeutic agent for psoriasis.

<u>BRIEF DESCRIPTION OF THE DRAWING</u>

FIG. 1 is a graph showing the effect of suppressing the proliferation of cultured human keratinocytes by an active vitamin $D_3$ (designated as "1,25D3" in the figure) and 2β-(3-hydroxypropyloxy)-1α,25-dihydroxyvitamin $D_3$ (designated as "ED-71" in the figure). In the figure, filled rhombuses (♦) represent the active vitamin $D_3$, and open circles (○) represent ED-71.

PREFERRED MODE FOR CARRYING OUT THE INVENTION

The entire disclosure of Japanese Patent Application No. 2002-224297, an application as the basis for priority claimed by the present application, is incorporated herein by reference in its entirety.

The compound represented by the Formula (I), namely, 2β-(3-hydroxypropyloxy)-1α,25-dihydroxyvitamin $D_3$, can be synthesized, for example, by the method described in JP 61-267549 A, although the method for its synthesis is not limited.

The therapeutic agent for psoriasis according to the present invention can be administered orally, parenterally (subcutaneous injection, intravenous injection, intramuscular injection, intraperitoneal injection, etc.), enterally, or topically. Topical administration, such as by an agent for external use, is preferred, but systemic administration as an oral agent or an injection may be performed. It is also possible to use a mode of administration, such as oral administration, injection, or external use, in a suitable combination.

The therapeutic agent for psoriasis according to the present invention may contain a pharmaceutically acceptable carrier or diluent in addition to the active ingredient. Examples of the carrier or diluent are vehicles (starch, lactose, etc.), disintegrants (alginic acid, etc.), tablet lubricants (stearic acid, talc, etc.), binders (starch, etc.), antioxidants (ascorbic acid, etc.), emulsifiers (polysorbate, etc.), surfactants (sorbitan monoesters, etc.), preservatives (benzoic acid), perfumes, and colorants. Other therapeutic ingredients may be contained further.

The therapeutic agent for psoriasis according to the present invention can be appropriately formulated according to the route of administration, such as oral administration, enteral administration, parenteral (including subcutaneous, intramuscular, and intravenous) administration, or external use.

For oral administration, such formulations as tablets, capsules, powders, granules, syrups, and elixirs are available. For parenteral administration, such formulations as injections (e.g., liquids or suspensions) are available. For external use as topical administration, such formulations as ointments, creams and lotions are available. For enteral administration, such formulations as suppositories and enemas are available.

The dose of the therapeutic agent for psoriasis in the present invention can be selected, as appropriate, according to the state of disease, the body weight and age of a subject to be treated, the route of administration and the dosage form of the agent of the present invention. For administration to animals, the dose is greatly affected by the body weight of individual animals. In the human adult, the usual oral dose of 2β-(3-hydroxypropyloxy)-1α,25-dihydroxyvitamin $D_3$, as the active ingredient, can be selected from the range of 0.0001 μg to 1,000 μg, preferably 0.001 μg to 100 μg, more preferably 0.01 μg to 10 μg, most preferably 0.1 μg to 1 μg, per day, and this dose can be used once daily or as two to three divided doses per day. For external medicine, such as an ointment, the dose of this compound as the active ingredient can be selected from the range of 0.0001 μg to 10,000 μg, preferably 0.001 μg to 1,000 μg, more preferably 0.01 μg to 100 μg, most preferably 0.1 μg to 25 μg, per day.

Examples

The present invention will be described in further detail by the following Examples and Manufacturing Examples.

(Example 1)

The effect of suppressing the proliferation of cultured human keratinocytes by 2β-(3-hydroxypropyloxy)-1α, 25-dihydroxyvitamin $D_3$ (hereinafter referred to as "ED-71") was investigated.

KGM-2 culture medium was added to each well of a 96-well plate (COSTAR 3595), and adult-human-derived keratinocytes (Clonetics) were seeded at a cell count of $1\times10^3$/well. Then, active vitamin $D_3$ (1α,25-dihydroxyvitamin $D_3$, produced by Solvay Pharmaceuticals) or ED-71 (produced by Chugai Seiyaku) was added to each well in a final concentration of $1\times10^{-10}$ mol/L, $1x10^{-9}$ mol/L, $1\times10^{-8}$ mol/L, or $1\times10^{-7}$ mol/L. The cells were cultured in the KGM-2 culture medium at a cell concentration of $1\times10^3$/200 μl/well for 3 days at 37°C in an atmosphere of 5% $CO_2$ and 95% air. [$^3$H]thymidine was added in an amount of 7.4 kBq/well, and the cells were further cultured for 1 day. The culture medium was removed, and the cells were stripped off using 0.05% trypsin/EDTA (GIBCO BRL), and the amount of [$^3$H]thymidine taken up by the cells was measured with a liquid scintillation counter (1450 MICROBETA, Wallac). The cells cultured and treated in the same manner as described above, except for the addition of the active vitamin $D_3$ or ED-71, were used as a control.

The results are shown in FIG. 1. In FIG. 1, the [$^3$H]thymidine uptake into the cells treated with each drug is expressed as a percentage of the [$^3$H]thymidine uptake into the control cells.

As shown in FIG. 1, the $IC_{50}$ (mol/L) value of the active vitamin $D_3$ was $3.05\times10^{-8}$ mol/L, while the $IC_{50}$ (mol/L) value of ED-71 was $<1.0\times10^{-10}$ mol/L.

In accordance with the following calculation equation, the human keratinocyte proliferation suppressing activity of ED-71 was calculated as a relative value with respect to the active vitamin $D_3$. This activity was found to be 305.23 or more.

$$\text{Relative value} = (\text{IC}_{50}\text{ value of active vitamin } D_3)/(\text{IC}_{50}\text{ value of ED-71})$$

This outcome shows that ED-71 has a very potent keratinocyte proliferation suppressing action, as compared with active vitamin $D_3$.

(Example 2)

The effect of ED-71 administered percutaneously and orally was investigated using hairless mice.

Percutaneous administration of a vitamin $D_3$ derivative in hairless mice was reported to cause hyperplasia of the epidermis (British Journal of Dermatology 1995; 132; 841-852). Following a single percutaneous dose of active vitamin $D_3$ (1α,25-dihydroxyvitamin $D_3$) and ED-71 administered to hairless mice, ED-71 thickened the epidermis in a lower dose than the dose of active vitamin $D_3$. When active vitamin $D_3$ and ED-71 were administered orally to hairless mice for 4 days, ED-71 thickened the epidermis in a lower dose than the dose of active vitamin $D_3$. These results suggested that ED-71, administered percutaneously or orally, would be effective.

(Preparation Example 1)

ED-71 (0.5 mg) is mixed with a hydrophilic ointment having the following formulation to obtain a hydrophilic ointment containing 0.5 μg of ED-71 per gram:

| | |
|---|---|
| White petrolatum | 250 g |
| Stearyl alcohol | 220 g |
| Propylene glycol | 120 g |
| Sodium lauryl sulfate | 15 g |
| Ethyl parahydroxybenzoate | 0.25 g |
| Propyl parahydroxybenzoate | 0.15 g |
| Purified water | appropriate amount |
| Total amount | 1000 g |

(Preparation Example 2)

ED-71 (1.0 mg) is dissolved in 60 g of a triglyceride of a middle chain fatty acid, and 30 mg of sorbic acid is added as a stabilizer. The mixture is processed in accordance with a conventional method using a gelatin shell soft capsule manufacturing machine to obtain soft capsules containing 1.0 μg of ED-71 per capsule.

<u>INDUSTRIAL APPLICARTLITY</u>

As described above, 2β-(3-hydroxypropyloxy)-1α,25-dihydroxyvitamin $D_3$ has an excellent keratinocyte proliferation suppressing action. The therapeutic agent of the present invention, comprising this compound as an active ingredient, is expected to be useful for treatment of psoriasis.

## Claims

**1.** A therapeutic agent for psoriasis, comprising a compound represented by the following Formula (I)

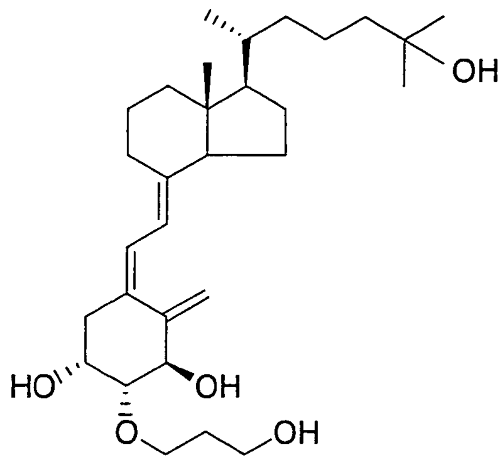

as an active ingredient.

**2.** The therapeutic agent for psoriasis according to claim 1, said therapeutic agent suppressing proliferation of keratinocytes.

**3.** A method for the treatment of psoriasis, comprising administering an effective amount of a compound represented by the following Formula (I)

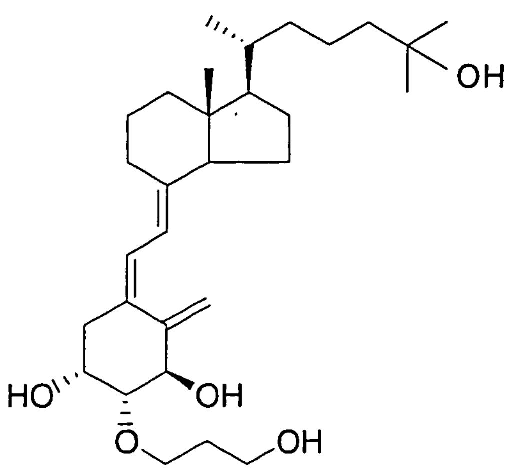

to a subject in need of such treatment.

**4.** The method according to claim 3, wherein proliferation of keratinocytes is suppressed.

*Fig. 1*

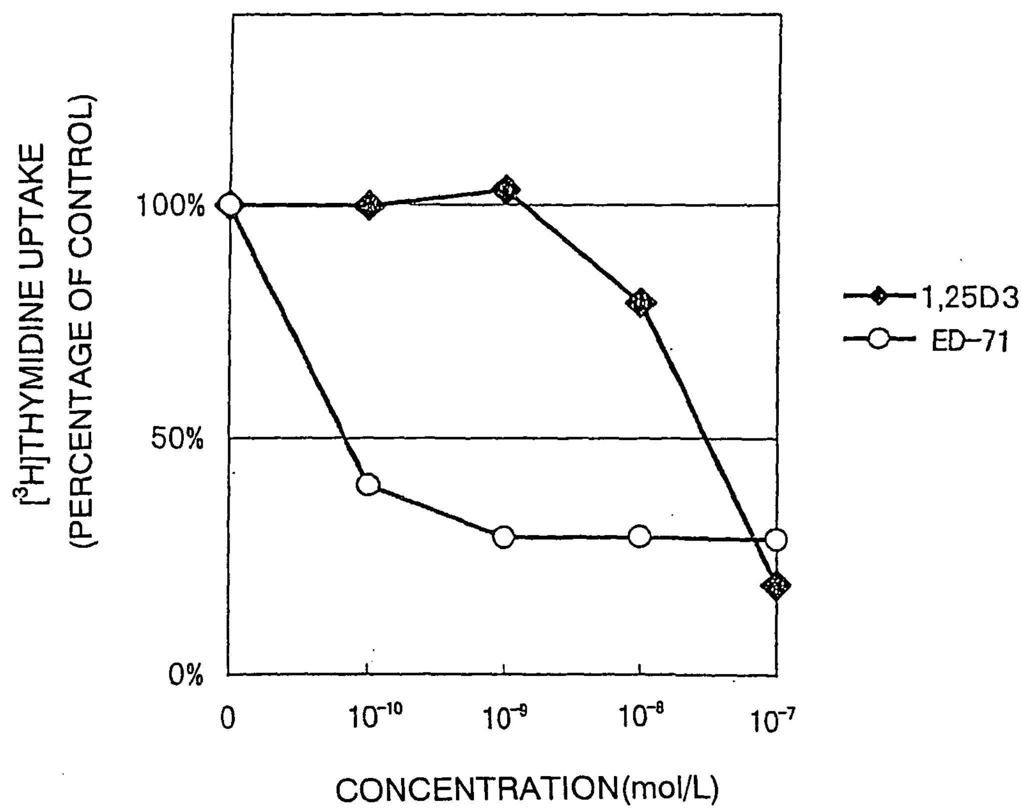
[3H]THYMIDINE UPTAKE
(PERCENTAGE OF CONTROL)
100%
50%
0%
0
10-10
10-9
10-8
10-7
CONCENTRATION(mol/L)
1,25D3
ED-71

**INTERNATIONAL SEARCH REPORT**

International application No.
PCT/JP03/09814

A. CLASSIFICATION OF SUBJECT MATTER
Int.Cl[7] A61K31/59, A61P17/06, A61P43/00

According to International Patent Classification (IPC) or to both national classification and IPC

B. FIELDS SEARCHED

Minimum documentation searched (classification system followed by classification symbols)
Int.Cl[7] A61K31/59, A61P17/06, A61P43/00

Documentation searched other than minimum documentation to the extent that such documents are included in the fields searched

Electronic data base consulted during the international search (name of data base and, where practicable, search terms used)
CAPLUS(STN), CAOLD(STN), REGISTRY(STN), BIOSIS(STN), MEDLINE(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| A | WO 96/00074 A1 (BENGURION UNIVERSITY OF THE NEGEV, KOST Joseph, SHANY Shraga, LAMPRECHT Sergio, SEGAL Garman), 04 January, 1996 (04.01.96), Claims 11, 14 & AU 9529980 B | 1,2 |
| A | JONES Glenville and CALVERLEY Martin J., 'A Dialogue on Analogues Newer Vitamin-D Drugs for Use in Bone Disease, Psoriasis, and Cancer' In: Trends in Endocrinology and Metabolism, Vol.4, No.9, 1993, pages 297 to 303 | 1,2 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>09 October, 2003 (09.10.03) | Date of mailing of the international search report<br>04 November, 2003 (04.11.03) |
| Name and mailing address of the ISA/<br>Japanese Patent Office<br>Facsimile No. | Authorized officer<br>Telephone No. |

Form PCT/ISA/210 (second sheet) (July 1998)

**INTERNATIONAL SEARCH REPORT**

International application No.
PCT/JP03/09814

**Box I Observations where certain claims were found unsearchable (Continuation of item 2 of first sheet)**

This international search report has not been established in respect of certain claims under Article 17(2)(a) for the following reasons:

1. [X] Claims Nos.: 3, 4
   because they relate to subject matter not required to be searched by this Authority, namely:
   Claims 3, 4 pertain to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.
2. [ ] Claims Nos.:
   because they relate to parts of the international application that do not comply with the prescribed requirements to such an extent that no meaningful international search can be carried out, specifically:
3. [ ] Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:
4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)